(19) Europäisches Patentamt
European Patent Office
Office européen des brevets

(11) EP 1 696 974 B1

(12) EUROPÄISCHE PATENTSCHRIFT

(45) Veröffentlichungstag und Bekanntmachung des Hinweises auf die Patenterteilung:
**23.05.2007 Patentblatt 2007/21**

(21) Anmeldenummer: **04804002.6**

(22) Anmeldetag: **17.12.2004**

(51) Int Cl.:
***A61L 15/60*** (2006.01) ***C08F 20/06*** (2006.01)
***C08F 20/56*** (2006.01) ***C08G 63/00*** (2006.01)

(86) Internationale Anmeldenummer:
**PCT/EP2004/014396**

(87) Internationale Veröffentlichungsnummer:
**WO 2005/061014 (07.07.2005 Gazette 2005/27)**

(54) **QUELLBARE HYDROGELBILDENDE POLYMERE MIT GERINGEM FEINSTAUBANTEIL**

SWELLABLE HYDROGEL-FORMING POLYMERS HAVING A LOW FINE DUST CONCENTRATION

POLYMERE GONFLABLE FORMANT UN HYDROGEL, A FAIBLE FRACTION FINE

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **19.12.2003 DE 10360394**
**03.02.2004 DE 102004005417**

(43) Veröffentlichungstag der Anmeldung:
**06.09.2006 Patentblatt 2006/36**

(73) Patentinhaber: **BASF Aktiengesellschaft**
**67056 Ludwigshafen (DE)**

(72) Erfinder:
- **RIEGEL, Ulrich**
**66849 Landstuhl (DE)**
- **DANIEL, Thomas**
**67165 Waldsee (DE)**
- **WEISMANTEL, Matthias**
**63637 Jossgrund (DE)**
- **ELLIOTT, Mark**
**67063 Ludwigshafen (DE)**
- **HERMELING, Dieter**
**67459 Böhl-Iggelheim (DE)**

(56) Entgegenhaltungen:
**WO-A-03/020978** **US-A- 5 275 838**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft quellbare hydrogelbildende Polymere mit geringem Feinstaubanteil, ein Verfahren zur Herstellung quellbarer hydrogelbildender Polymere mit geringem Feinstaubanteil sowie deren Verwendung.

Quellbare hydrogelbildende Polymere, sogenannte Superabsorber (Super-Absorbing Polymers, SAP), sind aus dem Stand der Technik bekannt.

Quellbare hydrogelbildende Polymere sind insbesondere Polymere aus (co)polymerisierten hydrophilen Monomeren, Pfropf(co)polymere von einem oder mehreren hydrophilen Monomeren auf einer geeigneten Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate. Solche Polymere werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau oder zur Verdickung aller Arten von Abfällen, insbesondere von medizinischen Abfällen, verwendet.

Quellbare hydrogelbildende Polymere sind bevorzugt solche mit einer Absorption von 0,9 gew.-%iger Kochsalzlösung von mindestens dem 10-fachen Eigengewicht bezogen auf das eingesetzte Polymer, bevorzugt dem 20-fachen Eigengewicht. Diese Absorption wird bevorzugt auch unter einem Druck beispielsweise von 0,7 psi erreicht.

Zur Verbesserung der Anwendungseigenschaften werden quellbare hydrogelbildende Polymere üblicherweise oberflächen- oder geinachvernetzt. Diese Nachvernetzung ist dem Fachmann an sich bekannt und erfolgt bevorzugt in wässriger Gelphase oder als Oberflächennachvernetzung der gemahlenen und abgesiebten Polymerpartikel.

Während der Herstellung und Verarbeitung von Superabsorbern kommt es durch abrasive Prozesse zur Abrundung der Partikel, wobei Feinstaub erzeugt wird. Der erzeugte Feinstaub führt zu Verstopfungen von Filtern, klebrigen Ablagerungen, Verklumpungen und erheblichen Schwierigkeiten bei der Förderung des Superabsorbers. Durch die große Oberfläche des Staubes kommt es zu schneller Feuchtigkeitsaufnahme aus der Umgebung und Verklebung des Niederschlags, was zur Verunreinigung der Produktionsanlagen führt. Innerhalb pneumatischer Fördersysteme bilden sich weiterhin harte Staubagglomerate. Weiterhin ist der feine Abrieb vom hygienischen und arbeitsmedizinischen Aspekt äußerst unerwünscht.

Der feine Staub führt, wenn er nicht speziell abgetrennt wird, entweder bei der Superabsorberherstellung oder vor dem Einbringen des Polymers in einen Hygieneartikel zu Problemen sowie zu einer stark verringerten Flüssigkeitsweiterleitung (SFC) des Gels bei der Anwendung. Dies führt dann zu erhöhten Leckraten.

Daher wurden bereits in den Patentanmeldungen WO-A-92/13912, WO-A-94/22940 und EP-A-0 679 678 Verfahren zur Entstaubung von Superabsorbern vorgeschlagen.

WO-A-92/13912 und WO-A-94/22940 beschreiben die Entstaubung von Superabsorbern durch oberflächliche Beschichtung mit Polyethylenglykolen. Polyethylenglykole haben den Nachteil, dass sie als lineare und wasserlösliche Polymere die Viskosität der die Gelpartikel umgebenden Lösung stark erhöhen und so deren Fliessfähigkeit verringern. Dies führt in der Anwendung im Hygieneartikel zu verschlechterter Flüssigkeitsweiterleitung (SFC) im gequollenen Gel.

EP-A-0 679 678 beschreibt ein Verfahren, bei dem der Staubanteil eines pulverförmigen Superabsorbers durch Nachbehandlung mit Silikonen gesenkt wird. Die Schrift empfiehlt auch die Verwendung zusätzlicher Entstaubungsmittel, wie beispielsweise Polyglykole und Polyglykolether. Silikone hydrophobieren den Superabsorber in unerwünschter Weise und verringern so die Quellgeschwindigkeit.

Weiterhin wird in der Patentanmeldung EP-A-0 755 964 die Oberflächenbeschichtung von Superabsorbern mit unlöslichen Wachsen beschrieben. Die verwendeten Wachse hydrophobieren den Superabsorber jedoch teilweise und sind nur schwer ohne Hilfsmittel dispergierbar. Die üblicherweise für diese Zwecke eingesetzten Dispergierhilfsmittel haben aber tensidischen Charakter und setzen die Oberflächenspannung der im Hygieneartikel eingeschlossenen Flüssigkeit herab, was wiederum zum Auslaufen führen kann.

Die Fixierung von Superabsorberpartikeln an Fasern unter Einsatz geeigneter polymerer und nicht-polymerer Bindemittel ist in den Patentschriften US 5,641,561 sowie US 5,589,256 beschrieben. Das Bindemittel soll in der Lage sein über Wasserstoffbrückenbindungen die Superabsorberpartikel an die Fasern im Hygieneartikel zu binden. Der unter Umständen negative Einfluss des Bindemittels auf die Flüssigkeitsleitfähigkeit des gequollenen Gels, insbesondere im Hinblick auf die offenbarten polymeren Bindemittel, oder die Oberflächenspannung der im Hygieneartikel eingeschlossenen Flüssigkeit wird weder erwähnt noch wird eine Lösung dieser Probleme offenbart. Hinsichtlich der nicht-polymeren Bindemittel ist auch hervorzuheben, dass diese als Lösemittel in Hygieneartikeln wegen der Ausdünstungen im Gebrauchszustand und der Wirkung auf die Haut nicht sehr erwünscht sind. Eine Lehre zur Minimierung solcher Lösemittel bei gleichzeitiger Entstaubung und Fixierung der Partikel an die Faser wird nicht offenbart.

Es bestand daher die Aufgabe ein Verfahren zur Herstellung von quellbaren hydrogelbildenden Polymeren, sogenannten Superabsorbern, zur Verfügung zu stellen, bei dem ein Superabsorber mit geringem Feinstaubanteil erhalten wird und bei dem weder das Quellverhalten noch die Flüssigkeitsweiterleitung gegenüber dem unbehandelten Superabsorber verschlechtert wird.

Eine weitere zu lösende Aufgabe war ein Verfahren zur Herstellung von quellbaren hydrogelbildenden Polymeren zur Verfügung zu stellen, bei dem ein Superabsorber erhalten wird, bei dem der Feinstaubanteil auch bei mechanischer Belastung nicht oder nur geringfügig ansteigt. Als Feinstaub werden Teilchen mit einem Durchmesser von weniger als 10 μm bezeichnet.

Eine weitere zu lösende Aufgabe war ein Verfahren zur Herstellung von Superabsorbern zur Verfügung zu stellen, bei dem die Superabsorber mit pulverförmigen und/oder staubförmigen Additiven nachbehandelt werden und bei dem die Superabsorber einen niedrigen Feinstaubanteil aufweisen.

Eine weitere zu lösende Aufgabe war ein Verfahren zur Herstellung von Superabsorbern zur Verfügung zu stellen, bei dem Superabsorber mit optimierten Förderverhalten erhalten werden. Dabei sollten die Superabsorber eine gewisse Klebrigkeit aufweisen, um beispielsweise problemlos mit Förderschnecken dosiert werden zu können, ohne das die Verbackungsneigung, insbesondere bei hoher Luftfeuchtigkeit, zunimmt.

Überraschend wurde nun gefunden, dass durch Verwendung hydrophiler Polymere mit dendritischer Struktur bei der Herstellung quellbarer hydrogelbildender Polymere Superabsorber mit geringem Feinstaubanteil, insbesondere auch nach mechanischer Belastung, einem verbesserten Bindevermögen an pulverförmige und/oder staubförmige Additive, hoher Anquellgeschwindigkeit, hoher Flüssigkeitsweiterleitung und optimalem Fließverhalten erhalten werden.

Dendritische Polymere sind nach Römpp, Lexikon-Chemie, Georg Thieme Verlag, Stuttgart, 10. Auflage, Seite 898, synthetische Makromoleküle, die durch schrittweise Verknüpfung von jeweils zwei oder mehr Monomeren mit jedem bereits gebundenen Monomeren aufgebaut werden, so dass mit jedem Schritt die Zahl der Monomer-Endgruppen exponentiell anwächst und am Ende eine kugelförmige Baumstruktur entsteht.

Erfindungsgemäß einsetzbare hydrophile Polymere mit dendritischer Struktur sind Polyole mit mindestens 8, vorzugsweise mindestens 16, besonders bevorzugt mindestens 32, Hydroxylgruppen und einem nichtlinearen, vorzugsweise mindestens 14fach verzweigten, besonders bevorzugt 30fach verzweigten, Grundgerüst.

Hydrophile Polymere mit dendritischer Struktur sind beispielsweise Polyester, die ausgehend von einem Polyol durch Veresterung mit einer $C_3$-$C_{20}$-Hydroxycarbonsäure, vorzugsweise mit einer $C_4$-$C_{12}$-Hydroxycarbonsäure, besonders bevorzugt mit einer $C_5$-$C_8$-Hydroxycarbonsäure, erhalten werden, wobei die Hydroxycarbonsäure mindestens zwei Hydroxylgruppen, vorzugsweise zwei Hydroxylgruppen, und/oder mindestens zwei Carbonsäuregruppen aufweist. Besonders bevorzugt sind Hydroxycarbonsäuren mit zwei Hydroxylgruppen und einer Carbonsäuregruppe, insbesondere 2,2-Dimethylolpropionsäure. Polyole sind Verbindungen mit mindestens zwei Hydroxylgruppen, wie beispielsweise Ethylenglykol, Diethylenglykol, Triethylenglykol, Polyethylenglykol, Propylenglykol, Dipropylenglykol, Tripropylenglykol, Polypropylenglykol, Butylenglykol, 1,3-Propandiol, 1,4-Butandiol, Bisphenol A, Glycerin, Trimethylolpropan, Pentaerythrit und/oder Sorbitol. Bevorzugt sind dentritische Polyester, besonders bevorzugt sind Boltorn® 20, Boltorn® 30, Boltorn® 40 und Boltorn® 310 (Perstorp Specialty Chemicals AB, SE).

Zu den erfindungsgemäß verwendbaren hydrophilen Polymeren mit dendritischer Struktur zählen auch Polymere, die durch Kondensation von Polyolen mit mindestens drei Hydroxylgruppen und anschließende Alkoxilierung zugänglich sind. Beispiele hierfür sind verzweigte Polyethylenglykole, die durch Kondensation von Glycerinmolekülen und anschließender Ethoxilierung zugänglich sind.

Weiterhin zählen zu den erfindungsgemäß verwendbaren hydrophilen Polymeren mit dendritischer Struktur aber auch alle Polymeren, die durch Polymerisation eines Monomeren mit mindestens einer Hydroxylgruppe und anschließende Alkoxilierung zugänglich sind. Vorzugsweise wird in Gegenwart eines Vernetzer polymerisiert. Dadurch werden Polymerpartikel erhalten, die an ihrer Oberfläche hydrophil sind und eine Vielzahl an Hydroxylgruppen aufweisen. Beispielsweise sind nach Makromol. Chem. 189, 2885 (1988) durch radikalische Polymerisation von p-Hydroxyethylstyrol und anschließende Alkoxilierung sogenannte Stern-Polyethylenglykole erhältlich.

Weitere Beispiele für erfindungsgemäß einsetzbare Polymere sind die hochverzweigten Polymere der Marke HYBRANE® sowie die Astramol-Dendrimere® (DSM N.V., NL). Hierzu zählen insbesondere hochverzweigte Poly(propylenimine), beispielsweise ausgehend von Butylendiamin durch wiederholte, mehrfache Michael-Addition mit Acrylnitril und Hydrierung erhältlich, Star-Polycaprolactone, Star-Nylon-6, hochverzweigte Polyesteramide, beispielsweise auf Basis des Additionsprodukts Bernsteinsäureanhydrid und Diethanolamin im Molverhältnis 1:1. Im erfindungsgemäßen Verfahren lassen sich auch sogenannte PAMAM-Dendrimere auf Basis Poly(amidoamin), beispielsweise ausgehend von Ammoniak durch wiederholte, mehrfache Umsetzung mit Methylacrylat und Ethylendiamin erhältlich, einsetzen. Einsetzbar sind Polyglycerole, sternförmige Polyethylenglykole sowie andere hydrophile Verbindungen, bevorzugt aber Polyalkohole, mit kugel- oder haufenförmiger, nicht jedoch linearer Molekulargeometrie.

Bevorzugt sind hydrophile Polymere mit dendritischer Struktur mit einer Glasübergangstemperatur Tg von 20 bis 100°C, besonders bevorzugt von 25 bis 50°C, und/oder einem mittleren Molgewicht von 1000 bis 10000 g/mol, besonders bevorzugt von 2000 bis 6000 g/mol.

Im erfindungsgemäßen Verfahren werden vorzugsweise 0,005 bis 10 Gew.-%, besonders bevorzugt 0,01 bis 5 Gew.-%, ganz besonders bevorzugt 0,05 bis 1 Gew.-%, insbesondere 0,10 bis 0,80 Gew.-%, hydrophiles Polymeres mit dendritischer Struktur bezogen auf das quellbare hydrogelbildende Polymer, eingesetzt.

Die hydrophilen Polymere mit dendritischer Struktur werden vorzugsweise mit dem getrockneten wasserabsorbierenden Hydrogel vermischt. Trocken bedeutet vorzugsweise einen Wassergehalt von weniger als 20 Gew.-%,

besonders bevorzugt von weniger als 10 Gew.-%. Das hydrophile Polymer mit dendritischer Struktur kann aber auch vor, während und/oder nach der Oberflächennachvernetzung dem quellbaren hydrogelbildende Polymer zugesetzt werden, vorzugsweise erfolgt die Auftragung aber während der Oberflächennachvernetzung.

Die Art des Mischens unterliegt keinen Beschränkungen, vorzugsweise werden Reaktionsmischer oder Misch- und Trocknungsanlagen, wie beispielsweise Lödige®-Mischer, BEPEX®-Mischer, NAUTA®-Mischer, SCHUGGI®-Mischer, NARA®-Trockner und PROCESSALL®, verwendet. Überdies können auch Wirbelschichttrockner eingesetzt werden. Die Mischung wird zweckmäßigerweise mit einer Verweilzeit von 1 bis 180 Minuten, vorzugsweise von 5 bis 20 Minuten, und einer Drehzahl von 25 bis 375 U/min, vorzugsweise von 100 bis 150 U/min, durchgeführt.

Bei Auftragung zusammen mit der Oberflächennachvernetzungslösung kann der Oberflächennachvernetzer zusammen mit dem dendritischen Polymer in Lösung gebracht werden, wahlweise können aber auch separate Flüssigkeitsströme in den Nachvernetzungsmischer über separate Düsen eingedüst werden. Bei Auftragung staub- oder pulverförmiger Additive ist es weiterhin möglich, dass man das dendritische Polymer in einem Lösemittel löst, in welchem auch das staub- oder pulverförmige Additiv dispergiert werden kann. Optional kann diese Mischung auch den Oberflächennachvemetzer enthalten.

Geeignete Lösemittel sind alle dem Fachmann bekannten Lösemittel, die in der Oberflächennachvernetzung Verwendung finden. Besonders bevorzugt ist Wasser, weiterhin bevorzugt sind Propandiol-1,2, Propandiol-1,3, Butandiol-1,4, Isopropanol, Ethanol,

Methanol, Ethylencarbonat, Propylencarbonat, Glycerin sowie Gemische untereinander. Besonders bevorzugt sind Gemische von Wasser mit einem oder mehreren der vorgenannten organischen Lösemittel. Die Wahl des Lösemittels ist jedoch nach den Erfordernissen gerichtet, die zu einer effektiven Herstellung der Lösung führen und nicht auf die vorgenannten eingeschränkt.

Optional kann dem Lösemittel auch ein oder mehrere oberflächenaktive Substanzen oder Dispergierhilfsmittel zugesetzt werden. Es können vorzugsweise nicht-ionische Tenside wie beispielsweise Sorbitanmonolaurat (Span 20), Sorbitanmonododecanat, Sorbitanmonohexadecanat, Sorbitanmonooctadecanat, Sorbitanmonooleat, Sorbitansesquioleat, Sorbitantrioleat welche unter den Handelsnamen Span 40, Span 60, Span 80, Span 83, Span 85 erhältlich sind, zugesetzt werden.

Vorzugsweise wird der Lösung jedoch keine oberflächenaktive Substanz als Dispergierhilfe zugesetzt.

Die Herstellung der Lösung oder Dispersion des dendritischen Polymers, die optional ein oder mehrere dispergierte staub- oder pulverförmige Additive enthalten kann, die optional ein Dispergierhilfsmittel enthalten kann, die weiterhin optional Aluminiumsulfat oder ein lösliches Metallsalz eines anderen 3- oder 4-valenten Metalls enthalten kann, und die optional mindestens einen Oberflächennachvernetzer enthalten kann, erfolgt bevorzugt durch Aufschmelzen -falls erfoderlich- des dendritischen Polymers und Eingiessen der Schmelze in das Lösemittel oder eines Teils des Lösemittels und anschliessender Verdünnung mit dem anderen Teil. Dabei wird vorzugsweise gut gerührt, bevorzugt turbulent gerührt z.B. mittels eines Ultraturax. Alternativ kann das dendritische Polymer auch direkt im heissen Lösemittel oder eines Teils davon aufgeschmolzen werden. Weiterhin kann man das dendritische Polymer beispielsweise auch mittels Ultraschall oder auch mit geeigneten Düsen dispergiern.

Sollen weiterhin staub- oder pulverförmige Additive eindispergiert werden, empfiehlt sich besonders die Verwendung eines geeigneten kontinuierlichen oder batch-Mischers zur Herstellung der Dispersion. Besonders bevorzugt sind Mischer der Firma IKA-Werke GmbH & Co KG des Typs MHD 2000/4, MHD 2000/5 sowie CMS 2000/4. Ähnlich aufgebaute Mischer, auch anderer Hersteller, können natürlich auch verwendet werden. Weiterhin können Mahlpumpen wie in DE 10131606 beschrieben wie z.B. NEWX 80-50-315, Fa. Wernert-Pumpen GmbH zur Herstellung der Dispersion verwendet werden.

In speziellen Fällen kann die Dispersion auch durch Beschallung mittels Ultraschall im Batch- oder Kontiverfahren hergestellt werden. Weiterhin können die üblichen konventionellen Nassmahlverfahren zur Herstellung der Dispersion verwendet werden. Besonders bevorzugt ist auch die nasschemische feinteilige Ausfällung durch chemische Reaktion zwischen löslichen Komponenten, gegebenenfalls unter Rühren unter Tempern, die in der Regel zu besonders feinteiligen Niederschlägen führt.

Ein weiterer Gegenstand der Erfindung sind quellbare hydrogelbildende Polymere, die nach dem erfindungsgemäßen Verfahren erhältlich sind, insbesondere quellbare hydrogelbildende Polymere mit einem Anteil an Partikeln mit einem Durchmesser von weniger als 10 $\mu$m von weniger als 100 Gew.-ppm, vorzugsweise von weniger als 50 Gew.-ppm, besonders bevorzugt von weniger als 10 Gew.-ppm, sowie deren Anwendung zur Absorption von Blut und/oder Körperflüssigkeiten, insbesondere von Urin.

Ein weiterer Gegenstand der Erfindung sind quellbare hydrogelbildende Polymere, die nach dem erfindungsgemäßen Verfahren erhältlich sind, insbesondere quellbare hydrogelbildende Polymere mit einem Anteil an Partikeln mit einem Durchmesser von weniger als 10 $\mu$m von weniger als 100 Gew.-ppm, vorzugsweise von weniger als 50 Gew.-ppm, besonders bevorzugt von weniger als 10 Gew.-ppm, wobei das quellbare hydrogelbildende Polymer mindestens ein pulverförmiges und/oder staubförmiges Additiv, wie beispielsweise Metallsalze, wie Aluminiumsulfat und/oder Magnesiumsulfat, pyrogene Kieselsäuren, wie Aerosil® 200, Polysaccharide und deren Derivate, nichtionische

Tenside, Wachse, Diatomeenerde und/oder Mikrohohlkugeln, enthält, sowie deren Anwendung zur Absorption von Blut und/oder Körperflüssigkeiten, insbesondere von Urin.

Mikrohohlkugeln werden in Chem. Ing. Techn. 75, 669 (2003) beschrieben. Mikrohohlkugeln sind gasgefüllte oder evakuierte globuläre Feststoffpartikel mit Durchmessern von 1 bis 1000 $\mu$m. Die typischen Wanddicken der Mikrohohlkugeln betragen zwischen 1 und 10 % des Durchmessers. Die Wandmaterialien unterliegen keiner Beschränkung. Mögliche Wandmaterialien sind Glas, keramikbildende Oxide oder Mischoxide, Silikate, Alumosilikate, Polymere, Polykondensate und Metalle.

Die erfindungsgemäßen quellbaren hydrogelbildenden Polymere haben typischerweise eine Flüssigkeitsweiterleitung (SFC) von mindestens 20 x $10^{-7}$ $cm^3s/g$, vorzugsweise mindestens 40 x $10^{-7}$ $cm^3s/g$, besonders bevorzugt mindestens 60 x $10^{-7}$ $cm^3s/g$, und noch mehr bevorzugt mindestens 150 x $10^{-7}$ $cm^3s/g$, und am meisten bevorzugt mindestens 300 x $10^{-7}cm^3s/g$. Ganz besonders bevorzugt erfindungsgemäss erhalten werden auch hydrogelbildende Polymere mit einer SFC von 500 - 2000 x $10^{-7}$ $cm^3s/g$..

Pulverförmige Additive haben vorzugsweise eine mittlere Teilchengröße von weniger als 2000 $\mu$m, besonders bevorzugt von weniger als 400$\mu$m.

Staubförmige Additive haben eine mittlere Teilchengröße von weniger als 200$\mu$m, vorzugsweise von weniger als 50 $\mu$m, besonders bevorzugt von weniger als 10 $\mu$m. Ein weiterer Gegenstand der Erfindung sind Hygieneartikel, welche erfindungsgemäß hergestellte Superabsorber enthalten.

Die im erfindungsgemäßen Verfahren einsetzbaren quellbaren hydrogelbildenden Polymere sind insbesondere Polymere aus vernetzten (co)polymerisierten hydrophilen Monomeren, Polyasparaginsäure, Pfropf(co)polymere von einem oder mehreren hydrophilen Monomeren auf eine geeignete Pfropfgrundlage, vernetzte Cellulose- oder Stärkeether oder in wässrigen Flüssigkeiten quellbare Naturprodukte, wie beispielsweise Guarderivate. Bevorzugt handelt es sich bei dem zu vernetzenden Polymer um ein Polymer, das Struktureinheiten enthält, die sich von Acrylsäure oder deren Estern ableiten, oder die durch Pfropfcopolymerisation von Acrylsäure oder Acrylsäureestern auf eine wasserlösliche Polymermatrix erhalten wurden. Diese Hydrogele sind dem Fachmann bekannt und beispielsweise in der US 4,286,082, DE-C-27 06 135,
US 4,340,706, DE-C-37 13 601, DE-C-28 40 010, DE-A-43 44 548,
DE-A-40 20 780, DE-A-40 15 085, DE-A-39 17 846, DE-A-38 07 289,
DE-A-35 33 337, DE-A-35 03 458, DE-A-42 44 548, DE-A-42 19 607,
DE-A-40 21 847, DE-A-38 31 261, DE-A-35 11 086, DE-A-31 18 172,
DE-A-30 28 043, DE-A-44 18 881, EP-A-0 801 483, EP-A-0 455 985,
EP-A-0 467 073, EP-A-0 312 952, EP-A-0 205 874, EP-A-0 499 774,
DE-A 26 12 846, DE-A-40 20 780, EP-A-0 205 674, US 5,145,906, EP-A-0 530 438, EP-A-0 670 073, US 4,057,521, US 4,062,817, US 4,525,527, US 4,295,987,
US 5,011,892, US 4,076,663 oder US 4,931,497 beschrieben. Der Inhalt der vorstehend genannten Patentdokumente ist betreffend Art und Herstellung solcher Hydrogele ausdrücklich Bestandteil der vorliegenden Offenbarung.

Zur Herstellung dieser quellbaren hydrogelbildenden Polymere geeignete hydrophile Monomere sind beispielsweise polymerisationsfähige Säuren, wie Acrylsäure, Methacrylsäure, Vinylsulfonsäure, Vinylphosphonsäure, Maleinsäure einschließlich deren Anhydrid, Fumarsäure, Itaconsäure, 2-Acrylamido-2-methylpropansulfonsäure, 2-Acrylamido-2-methylpropanphosphonsäure sowie deren Amide, Hydroxyalkylester und aminogruppen- oder ammoniumgruppenhaltige Ester und Amide sowie die Alkalimetall- und/oder Ammoniumsalze der Säuregruppen enthaltenden Monomeren. Des weiteren eignen sich wasserlösliche N-Vinylamide wie N-Vinylformamid oder auch Diallyldimethyl-ammoniumchlorid. Bevorzugte hydrophile Monomere sind Verbindungen der allgemeinen Formel I

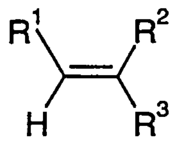

(I)

worin

$R^1$ Wasserstoff, $C_1$-$C_4$-Alkyl, wie beispielsweise Methyl oder Ethyl, oder Carboxyl,

$R^2$ -$COOR^4$, Hydroxysulfonyl oder Phosphonyl, eine mit einem $C_1$-$C_4$-Alkanol veresterte Phosphonylgruppe oder eine Gruppe der Formel II

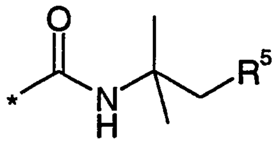

(II)

$R^3$ Wasserstoff, $C_1$-$C_4$-Alkyl, wie beispielsweise Methyl oder Ethyl,

$R^4$ Wasserstoff, $C_1$-$C_4$-Aminoalkyl, $C_1$-$C_4$-Hydroxyalkyl, Alkalimetall- oder Ammoniumion und

$R^5$ eine Sulfonylgruppe, eine Phosphonylgruppe oder eine Carboxylgruppe oder jeweils deren Alkalimetall- oder Ammoniumsalze, bedeuten.

Beispiele für $C_1$-$C_4$-Alkanole sind Methanol, Ethanol, n-Propanol, Isopropanol oder n-Butanol.

Besonders bevorzugte hydrophile Monomere sind Acrylsäure und Methacrylsäure, sowie deren Alkalimetall- oder Ammoniumsalze, beispielsweise Natriumacrylat, Kaliumacrylat oder Ammoniumacrylat.

Geeignete Pfropfgrundlagen für hydrophile Hydrogele, die durch Pfropfcopolymerisation olefinisch ungesättigter Säuren oder ihrer Alkalimetall- oder Ammoniumsalze erhältlich sind, können natürlichen oder synthetischen Ursprungs sein. Beispiele sind Stärke, Cellulose oder Cellulosederivate sowie andere Polysaccharide und Oligosaccharide, Polyalkylenoxide, insbesondere Polyethylenoxide und Polypropylenoxide, sowie hydrophile Polyester.

Geeignete Polyalkylenoxide haben beispielsweise die Formel III

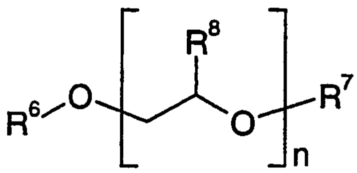

(III)

worin

$R^6$, $R^7$ unabhängig voneinander Wasserstoff, $C_1$-$C_{12}$-Alkyl, wie beispielsweise Methyl Ethyl, n-Propyl oder Isopropyl, $C_2$-$C_{12}$-Alkenyl, wie beispielsweise Ethenyl, n-Propenyl oder Isopropenyl, $C_7$-$C_{20}$-Aralkyl, wie beispielsweise Phenylmethyl, 1-Phenylethyl oder 2-Phenylethyl, oder Aryl, wie beispielsweise 2-Methylphenyl, 4-Methylphenyl oder 4-Ethylphenyl,

$R^8$ Wasserstoff oder Methyl und

n eine ganze Zahl von 1 bis 10000 bedeuten.

$R^6$ und $R^7$ bedeuten bevorzugt Wasserstoff, $C_1$-$C_4$-Alkyl, $C_2$-$C_6$-Alkenyl oder Phenyl.

Bevorzugte Hydrogele sind insbesondere Polyacrylate, Polymethacrylate sowie die in der US 4,931,497, US 5,011,892 und US 5,041,496 beschriebene Pfropfpolymere.

Die quellbaren hydrogelbildenden Polymere sind bevorzugt vernetzt, d.h. sie enthalten Verbindungen mit mindestens zwei Doppelbindungen, die in das Polymernetzwerk einpolymerisiert sind. Geeignete Vernetzer sind insbesondere N,N'-Methylenbisacrylamid und N,N'-Methylenbismethacrylamid, Ester ungesättigter Mono- oder Polycarbonsäuren von Polyolen, wie Diacrylat oder Triacrylat, beispielsweise Butandiol- oder Ethylenglykoldiacrylat bzw. -methacrylat sowie Trimethylolpropantriacrylat und Allylverbindungen wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP-A-0 343 427 beschrieben sind. Weiterhin einsetzbar im erfindungsgemäßen Verfahren sind auch Hydrogele, die unter Verwendung von Polyallylethern als Vernetzer und durch saure Homopolymerisation von Acrylsäure hergestellt werden. Geeignete Vernetzer sind Pentaerythritoltri- und -tetraallylether, Polyethylenglykoldiallylether, Ethylenglykoldiallylether, Glyceroldi- und Triallylether, Polyallylether auf Basis Sorbitol,

sowie ethoxilierte Varianten davon.

Die bevorzugten Herstellverfahren für das im erfindungsgemäßen Verfahren einsetzbare Basispolymer werden in "Modern Superabsorbent Polymer Technology", F.L. Buchholz and A.T. Graham, Wiley-VCH, 1998, Seiten 77 bis 84 beschrieben. Besonders bevorzugt sind Basispolymere, die im Kneter, wie beispielsweise in WO-A-01/38402 beschrieben, oder auf einem Bandreaktor, wie beispielsweise in EP-A-0 955 086 beschrieben, hergestellt werden.

Das wasserabsörbierende Polymer ist bevorzugt eine polymere Acrylsäure oder ein Polyacrylat. Die Herstellung dieses wasserabsorbierenden Polymeren kann nach einem aus der Literatur bekannten Verfahren erfolgen. Bevorzugt sind Polymere, die vernetzende Comonomere in Mengen von 0,001 bis 10 mol-%, vorzugsweise 0,01 bis 1 mol-% enthalten, ganz besonders bevorzugt sind jedoch Polymere, die durch radikalische Polymerisation erhalten wurden und bei denen ein mehrfunktioneller ethylenisch ungesättigter Radikalvernetzer verwendet wurde, der zusätzlich noch mindestens eine freie Hydroxylgruppe trägt (wie beispielsweise Pentaerythritoltriallylether oder Trimethylolpropandiallylether).

Die quellbaren hydrogelbildenden Polymere können durch an sich bekannte Polymerisationsverfahren hergestellt werden. Bevorzugt ist die Polymerisation in wässriger Lösung nach dem Verfahren der sogenannten Gelpolymerisation. Dabei werden beispielsweise 15 bis 50 gew.-%ige wässrige Lösungen eines oder mehrerer hydrophiler Monomere und gegebenenfalls einer geeigneten Pfropfgrundlage in Gegenwart eines Radikalinitiators, bevorzugt ohne mechanische Durchmischung unter Ausnutzung des Trommsdorff-Norrish-Effektes (Makromol. Chem. 1, 169 (1947)), polymerisiert. Die Polymerisationsreaktion kann im Temperaturbereich zwischen 0 und 150°C, vorzugsweise zwischen 10 und 100°C, sowohl bei Normaldruck als auch unter erhöhtem oder erniedrigtem Druck durchgeführt werden. Wie üblich kann die Polymerisation auch in einer Schutzgasatmosphäre, vorzugsweise unter Stickstoff, ausgeführt werden. zur Auslösung der Polymerisation können energiereiche elektromagnetische Strahlen oder die üblichen chemischen Polymerisationsinitiatoren herangezogen werden, beispielsweise organische Peroxide, wie Benzoylperoxid, tert.-Butylhydroperoxid, Methylethylketonperoxid, Cumolhydroperoxid, Azoverbindungen wie Azodiisobutyronitril sowie anorganische Peroxoverbindungen wie $(NH_4)_2S_2O_8$ oder $K_2S_2O_8$ oder $H_2O_2$. Sie können gegebenenfalls in Kombination mit Reduktionsmitteln wie Natriumhydrogensulfit und Eisen(II)-sulfat oder Redoxsystemen, welche als reduzierende Komponente eine aliphatische und aromatische Sulfinsäure, wie Benzolsulfinsäure und Toluolsulfinsäure oder Derivate dieser Säuren enthalten, wie Mannichaddukte aus Sulfinsäuren, Aldehyden und Aminoverbindungen, wie sie in der DE-A-13 01 566 beschrieben sind, verwendet werden. Durch mehrstündiges Nachheizen der Polymergele im Temperaturbereich 50 bis 130°C, vorzugsweise 70 bis 100°C, können die Qualitätseigenschaften der Polymere noch verbessert werden.

Die erhaltenen Gele werden beispielsweise zu 0 bis 100 mol-%, bevorzugt 5 und 90 mol-%, insbesondere zwischen 25 und 80 mol%, ganz besonders bevorzugt zwischen 30 und 55 mol-% und zwischen 70 und 75 mol-%, bezogen auf eingesetztes Monomer neutralisiert, wobei die üblichen Neutralisationsmittel verwendet werden können, bevorzugt Alkalimetallhydroxide oder -oxide, besonders bevorzugt jedoch Natriumhydroxid, Natriumcarbonat und Natriumhydrogencarbonat.

Üblicherweise wird die Neutralisation durch Einmischung des Neutralisationsmittels als wässrige Lösung oder bevorzugt auch als Feststoff erreicht. Das Gel wird hierzu mechanisch zerkleinert, beispielsweise mittels eines Fleischwolfes und das Neutralisationsmittel wird aufgesprüht, übergestreut oder aufgegossen, und dann sorgfältig untergemischt. Dazu kann die erhaltene Gelmasse noch mehrmals zur Homogenisierung gewolft werden. Die neutralisierte Gelmasse wird dann mit einem Band- oder Walzentrockner getrocknet bis der Restfeuchtegehalt vorzugsweise unter 10 Gew.-%, insbesondere unter 5 Gew.-% liegt. Das getrocknete Hydrogel wird hiernach gemahlen und gesiebt, wobei zur Mahlung üblicherweise Walzenstühle, Stiftmühlen oder Schwingmühlen eingesetzt werden können. Die Partikelgröße des gesiebten Hydrogels liegt vorzugsweise im Bereich 45 bis 1000 $\mu$m, besonders bevorzugt bei 45-850 $\mu$m, ganz besonders bevorzugt bei 100 bis 800 $\mu$m und noch mehr bevorzugt bei 100 bis 700 $\mu$m. Weitere bevorzugte Korngrössen liegen im Bereich 100 - 500 $\mu$m, 300 - 600 $\mu$m, kleiner als 600 $\mu$m, kleiner als 400 $\mu$m, besonders bevorzugt kleiner als 300 $\mu$m, und am meisten bevorzugt kleiner als 150 $\mu$m. In diesen Bereichen liegen mindestens 80%, bevorzugt mindestens 90% aller Partikel.

Die Nachvernetzung von quellbaren hydrogelbildenden Polymeren wird in der Regel so durchgeführt, dass eine Lösung des Oberflächennachvernetzers auf das trockene Grundpolymerpulver aufgesprüht wird. Im Anschluss an das Aufsprühen wird das Polymerpulver thermisch getrocknet, wobei die Vernetzungsreaktion sowohl vor als auch während der Trocknung stattfinden kann.

Bevorzugt ist das Aufsprühen einer Lösung des Vernetzers in Reaktionsmischem oder Misch- und Trocknungsanlagen, wie beispielsweise Lödige®-Mischer, BEPEX®-Mischer, NAUTA®-Mischer, SCHUGGI®-Mischer, NARA®-Trockner und PROCESSALL®. Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Es kann aber auch beispielsweise eine azeotrope Destillation als Trocknungsverfahren benutzt werden.

Bevorzugte Trocknungstemperaturen liegen im Bereich 50 bis 250°C, bevorzugt bei 60 bis 200°C, und besonders bevorzugt bei 70 bis 180°C. Die bevorzugte Verweilzeit bei dieser Temperatur im Reaktionsmischer oder Trockner beträgt unter 60, bevorzugt unter 30 Minuten, besonders bevorzugt unter 10 Minuten.

Die Oberflächennachvernetzer können allein oder in Kombination mit anderen Oberflächennachvernetzern verwendet werden, beispielsweise Ethylenglykoldiglycidylether, Diethylenglykoldiglycidylether, Polyethylenglykoldiglycidylether, Propylenglykoldiglycidylether, Dipropylenglykoldiglycidylether, Polypropylenglykoldiglycidylether, Glycerindiglycidylether, Polyglycerindiglycidylether, Epichlorhydrin, Etylendiamin, Ethylenglykol, Diethylenglykol, Triethylenglykol, Polyethylenglykol, Propylenglykol, Dipropylenglykol, Tripropylenglykol, Polypropylenglykol, Butylenglykol, 1,3-Propandiol, 1,4-Butandiol,

Bisphenol A, Glycerin, Trimethylolpropan, Pentaerythrit, Sorbitol, Diethanolamin, Triethanolamin, Etylendiamin, Ethylencarbonat, Propylencarbonat, 2-Oxazolidone, wie 2-Oxazolidinon oder N-Hydroxyethyl-2-oxazolidinon, Morpholin-2,3-dione, wie N-2-Hydroxyethyl-morpholin-2,3-dion, N-Methyl-morpholin-2,3-dion, N-Ethyl-morpholin-2,3-dion und/oder N-tert.-Butyl-morpholin-2,3-dion, 2-Oxotetrahydro-1,3-oxazin, N-Acyl-2-oxazolidone, wie N-Acetyl-2-oxazolidon, bicyclische Amidacetale, wie 5-Methyl-1-aza-4,6-dioxa-bicyclo[3.3.0]octan, 1-Aza-4,6-dioxa-bicyclo[3.3.0]octan und/oder 5-Isopropyl-1-aza-4,6-dioxa-bicyclo[3.3.0]octan, und/oder Bis- und Poly-2-oxazolidinone.

Der Oberflächennachvernetzer wird bevorzugt in nicht selbst-reaktiven Lösemitteln gelöst, bevorzugt in niederen Alkoholen, wie beispielsweise Methanol, Ethanol, Isopropanol, Propylenglykol, Ethylenglykol, vorzugsweise Isopropanol, ganz besonders bevorzugt in wässrigen Lösungen solcher geeigneter Alkohole, wobei der Alkoholgehalt der Lösung 10 bis 90 Gew.-%, besonders bevorzugt zwischen 25 bis 70 Gew.-%, insbesondere zwischen 30 bis 50 Gew.-% beträgt.

Der Oberflächennachvernetzer wird dabei in einer Menge von 0,01 bis 1 Gew.-%, bezogen auf das eingesetzte Polymer, verwendet, und die Vernetzerlösung selbst in einer Menge von 1 bis 20 Gew.-%, bevorzugt 3 bis 15 Gew.-%, bezogen auf das eingesetzte Polymer, verwendet.

Der AUL-0,7psi-Wert [g/g] der erfindungsgemäßen quellbaren hydrogelbildenden Polymere kann nach der in DE-A-199 09 653 angegebenen Methode gemessen werden und ist bevorzugt größer 10, insbesondere größer 15, besonders bevorzugt größer 20, insbesondere größer 25, insbesondere bevorzugt größer 30.

Die erfindungsgemäßen quellbaren hydrogelbildenden Polymere eignen sich zur Absorption von Blut und/oder Körperflüssigkeiten in Hygieneartikeln, wie beispielsweise Inkontinenzartikel, Binden, Tampons, Einlagen. Dazu können die erfindungsgemäßen quellbaren hydrogelbildenden Polymere mit Fasern, wie beispielsweise Zellulose, sowie Faservlies zu absorbierenden Verbundstoffen verarbeitet werden.

Die im erfindungsgemäßen Verfahren eingesetzten dendritischen Polymere sind aufgrund ihrer nichtlinearen Struktur zwar hydrophil, jedoch ist wegen der speziellen Geometrie die unerwünschte Fähigkeit zur thermischen Nachvernetzung stark eingeschränkt, so dass die dendritischen Polymere während der Oberflächennachvernetzung zugesetzt werden können. Ein zusätzlicher Einmischschritt ist nicht erforderlich. Besonders vorteilhaft ist dabei die globuläre Form hinsichtlich der Viskosität der wässrigen Lösung in angequollenen bzw. gequollenen Superabsorber. Dies hat zur Folge, dass die Flüssigkeitsweiterleitung unverändert hoch bleibt, auch bei hohem Polymereinsatz. Das Staubbindevermögen des dendritischen Polymeren ist hervorragend, insbesondere das Bindevermögen staubförmiger bzw. pulverförmiger Additive. Sowohl direkt nach dem Auftrag als auch nach harter mechanischer Beanspruchung ist praktisch kaum Feinstaub im Produkt nachweisbar.

Die Fördereigenschaften des Endprodukts werden auch durch das bei der Oberflächennachvernetzung verwendete Lösungsmittel beeinflusst. Dabei hat Propylenglykol/Wasser gegenüber Isopropanol/Wasser deutliche Vorteile. Andererseits lässt sich nicht umgesetztes Propylenglykol im Gegensatz zu nicht umgesetzten Isopropanol nur schlecht entfernen und verbleibt im Endprodukt. Der Alkoholgehalt im getrockneten Endprodukt beträgt typischerweise bei der Verwendung von Propylenglykol von 5000 bis 15000 Gew.-ppm und bei der bevorzugten Verwendung von Isopropanol weniger als 1000 Gew.-ppm, vorzugsweise weniger als 500 Gew.-ppm, besonders bevorzugt weniger als 100 Gew.-ppm.

Der Zusatz dendritischer Polymere im erfindungsgemäßen Verfahren ermöglicht die Verwendung von Isopropanol/Wasser (30 Gew.-% Isopropanol in Wasser) als Lösungsmittel bei der Oberflächennachvernetzung um Superabsorber mit Fördereigenschaften zu erhalten, die bislang nur bei Verwendung von Propylenglykol/Wasser (30 Gew.-% Propylenglykol in Wasser) zugänglich waren.

Zur Bestimmung der Güte der erfindungsgemäßen Nachbehandlung wird das getrocknete Hydrogel mit den Testmethoden geprüft, die nachfolgend beschrieben sind:

Methoden:

Die Messungen sollten, wenn nicht anders angegeben, bei einer Umgebungstemperatur von 23 ± 2°C und einer relativen Luftfeuchte von 50 ± 10 % durchgeführt werden. Das Quellbare hydrogelbildende Polymer wird vor der Messung gut durchmischt.

Zentrifugenretentionskapazität (CRC Centrifuge Retention Capacity)

Bei dieser Methode wird die freie Quellbarkeit des Hydrogels im Teebeutel bestimmt. Zur Bestimmung der CRC werden 0,2000 ± 0,0050 g getrocknetes Hydrogel (Kornfraktion 106 - 850 μm) in einem 60 x 85 mm großen Teebeutel eingewogen, der anschließend verschweißt wird. Der Teebeutel wird für 30 Minuten in einen Überschuss von 0,9 gew.-%iger Kochsalzlösung gegeben (mindestens 0,83 l Kochsalzlösung/1 g Polymerpulver). Anschließend wird der Teebeutel 3 Minuten lang bei 250 G zentrifugiert. Die Bestimmung der vom Hydrogel festgehaltenen Flüssigkeitsmenge geschieht durch Auswägen des zentrifugierten Teebeutels.

Die Zentrifugenretentionskapazität kann auch nach der von der EDANA (European Disposables and Nonwovens Association) empfohlenen Testmethode Nr. 441.2-02 "Centrifuge retention capacity" bestimmt werden.

Absorption unter Druck (AUL Absorbency Under Load) 0,7 psi (4830 Pa)

Die Messzelle zur Bestimmung der AUL 0,7 psi ist ein Plexiglas-Zylinder mit einem Innendurchmesser von 60 mm und einer Höhe von 50 mm, der an der Unterseite einen angeklebten Edelstahl-Siebboden mit einer Maschenweite von 36μm besitzt. Zu der Messzelle gehört weiterhin eine Plastikplatte mit einem Durchmesser von 59 mm und ein Gewicht, welches zusammen mit der Plastikplatte in die Messzelle hineingestellt werden kann. Das Gewicht der Plastikplatte und das Gewicht betragen zusammen 1344 g. Zur Durchführung der Bestimmung der AUL 0,7 psi wird das Gewicht des leeren Plexiglas-Zylinders und der Plastikplatte ermittelt und als $W_0$ notiert. Dann werden 0,900 ± 0,005 g quellbares hydrogelbildendes Polymer (Korngrößenverteilung 150 - 800 μm) in den Plexiglas-Zylinder eingewogen und möglichst gleichmäßig auf dem Edelstahl-Siebboden verteilt. Anschließend wird die Plastikplatte vorsichtig in den Plexiglas-Zylinder hineingelegt und die gesamte Einheit gewogen; das Gewicht wird als $W_a$ notiert. Nun wird das Gewicht auf die Plastikplatte in dem Plexiglas-Zylinder gestellt. In die Mitte der Petrischale mit einem Durchmesser von 200 mm und einer Höhe von 30 mm wird eine keramische Filterplatte mit einem Durchmesser von 120 mm und einer Höhe von 10 mm (Duran, Fa. Schott) und einer Porosität 0 gelegt und soviel 0,9 gew.-%ige Natriumchloridlösung eingefüllt, dass die Flüssigkeitsoberfläche mit der Filterplattenoberfläche abschließt, ohne dass die Oberfläche der Filterplatte benetzt wird. Anschließend wird ein rundes Filterpapier mit einem Durchmesser von 90 mm und einer Porengröße < 20 μm (S&S 589 Schwarzband von Schleicher & Schüll) auf die keramische Platte gelegt. Der quellbares hydrogelbildendes Polymer enthaltende Plexiglas-Zylinder wird mit Plastikplatte und Gewicht nun auf das Filterpapier gestellt und dort für 60 Minuten belassen. Nach dieser Zeit wird die komplette Einheit aus der Petrischale vom Filterpapier herausgenommen und anschließend das Gewicht aus dem Plexiglas-Zylinder entfernt. Der gequollenes Hydrogel enthaltende Plexiglas-Zylinder wird zusammen mit der Plastikplatte ausgewogen und das Gewicht als $W_b$ notiert.

Die Absorption unter Druck (AUL) wird wie folgt berechnet:

$$\text{AUL 0,7 psi [g/g]} = [W_b\text{-}W_a]/[W_a\text{-}W_0]$$

Die Absorption unter Druck kann auch nach der von der EDANA (European Disposables and Nonwovens Association) empfohlenen Testmethode Nr. 442.2-02 "Absorption under pressure" bestimmt werden.

Flüssigkeitsweiterleitung (SFC Saline Flow Conductivity)

Die Flüssigkeitsweiterleitung einer gequollenen Gelschicht unter Druckbelastung von 0,3 psi (2070 Pa) wird, wie in EP-A-0 640 330 beschrieben, als Gel-Layer-Permeability einer gequollenen Gelschicht aus superabsorbierendem Polymer bestimmt, wobei die in zuvor genannter Patentanmeldung auf Seite 19 und in Figur 8 beschriebene Apparatur dahingehend modifiziert wurde, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP-A-0 640 330. Der Durchfluss wird automatisch erfasst.

Die Flüssigkeitsweiterleitung (SFC) wird wie folgt berechnet:

$$\text{SFC [cm}^3\text{s/g]} = (F_g(t{=}0)xL_0)/(dxAxWP),$$

wobei $F_g(t{=}0)$ der Durchfluss an NaCl-Lösung in g/s ist, der anhand einer linearen Regressionsanalyse der Daten $F_g(t)$ der Durchflussbestimmungen durch Extrapolation gegen t=0 erhalten wird, $L_0$ die Dicke der Gelschicht in cm, d die

Dichte der NaCl-Lösung in $g/cm^3$, A die Fläche der Gelschicht in $cm^2$ und WP der hydrostatische Druck über der Gelschicht in $dyn/cm^2$ darstellt.

Fließgeschwindigkeit (FLR Flow Rate)

Bei der Methode wird die Geschwindigkeit ermittelt, mit der das quellbare hydrogelbildende Polymer durch einen Trichter fließt. Zur Bestimmung der FLR werden 100 ± 0,01 getrocknetes Hydrogel in einen verschließbaren Metalltrichter eingewogen. Das Gewicht des quellbaren hydrogelbildenden Polymeren wird als $W_1$ notiert. Der Trichter entspricht DIN 53492. Das Auslaufrohr des Trichters hat eine Höhe von 145,0 ± 0,5 mm und einen Innendurchmesser von 10,00 ± 0,01 mm. Der Neigungswinkel der Trichterwand gegenüber der Horizontalen beträgt 20°. Der Metalltrichter wird geerdet. Anschließend wird der Trichter geöffnet und die Zeit gemessen bis der Trichter entleert ist. Die Zeit wird als t notiert.

Die Messung wird doppelt durchgeführt. Die Abweichung beider Messwerte darf maximal 5 % betragen.

Die Fließgeschwindigkeit (FLR) wird wie folgt berechnet:

$$FLR\ [g/s] = W_1/t$$

Die Fließgeschwindigkeit kann auch nach der von der EDANA (European Disposables and Nonwovens Association) empfohlenen Testmethode Nr. 450.2-02 "Flowrate" bestimmt werden.

Ausschüttgewicht (ASG)

Bei der Methode wird die Dichte des quellbaren hydrogelbildenden Polymeren nach dem Ausschütten ermittelt. Die Messung wird mit einem zylindrischen Pygnometer entsprechend DIN 53466 durchgeführt. Das Pygnometer hat ein Volumen (V) von 100,0 ± 0,5 ml, einen Innendurchmesser von 45,0 ± 0,1 mm und eine Höhe von 63,1 ± 0,1 mm. Das Pygnometer wird leer gewogen. Das Gewicht wird als $W_1$ notiert. Zur Bestimmung des ASG werden ca. 100 g getrocknetes Hydrogel in einen verschließbaren Metalltrichter eingewogen. Der Trichter entspricht DIN 53492. Das Auslaufrohr des Trichters hat eine Höhe von 145,0 ± 0,5 mm und einen Innendurchmesser von 10,00 ± 0,01 mm. Der Neigungswinkel der Trichterwand gegenüber der Horizontalen beträgt 20°. Der Metalltrichter und das Pygnometer werden geerdet. Anschließend wird der Trichter in das Pynometer entleert, wobei überschüssiges quellbares hydrogelbildendes Polymer überläuft. Das überstehende quellbare hydrogelbildende Polymer wird mittels eines Spatels abgestrichen. Das gefüllte Pygnometer wird gewogen und das Gewicht als $W_2$ notiert.

Die Messung wird doppelt durchgeführt. Die Abweichung beider Messwerte darf maximal 5% betragen.

Das Ausschüttgewicht (ASG) wird wie folgt berechnet:

$$ASG\ [g/ml] = [W_2\text{-}W_1]/V$$

Das Ausschüttgewicht kann auch nach der von der EDANA (European Disposables and Nonwovens Association) empfohlenen Testmethode Nr. 460.2-02 "Density" bestimmt werden.

Bruchtest (Fragility test)

Mit dem Bruchtest wird das Verhalten des quellbaren hydrogelbildenden Polymeren bei mechanischer Belastung ermittelt. Der Test wird mit einem verschließbaren Steinzeugbecher durchgeführt. Der Steinzeugbecher hat einen Durchmesser von 85,7 mm (3 3/8 inch), eine Höhe von 111,1 mm (4 3/8 inch) und ein Volumen von 379 $cm^3$ (0,1 gallons). Die Öffnung hat einen Durchmesser von 31,8 mm (1 1/4 inch). Die Höhe mit Deckel beträgt 139,7 mm (5 1/2 inch). In den Steinzeugbecher werden 50 g quellbares hydrogelbildendes Polymer und 127 g zylindrische Steinzeugmahlkörper eingefüllt. Die Steinzeugmahlkörper haben einen Durchmesser von 12,7 mm (1/2 inch), eine Höhe von 12,7 mm (1/2 inch) und ein Einzelgewicht von ca. 5,3 g. Der Steinzeugbecher wird verschlossen und 15 Minuten auf einer Rollmühle mit Walzenstuhl (beispielsweise von der Fa. U.S. Stoneware, US) mit 180 U/min gedreht.

Staubanteile (Dust)

Die Staubanteile können nach der von der EDANA (European Disposables and Nonwovens Association) empfohlenen Testmethode Nr. 490.2-02 "Dust" bestimmt werden.

Verbackungstest (Anti-Caking)

In ein 100 ml Becherglas werden 30 g quellbares hydrogelbildendes Polymer eingewogen. Anschließend wird das Becherglas 2 Stunden bei 40°C und einer relativen Luftfeuchtigkeit von 95% gelagert. Nach der Lagerung wird das quellbare hydrogelbildende Polymer ausgeschüttet. Das Ausschüttverhalten wird qualitativ bewertet, dabei bedeutet "sehr schlecht", dass sich auf der Oberfläche des quellbare hydrogelbildenden Polymers eine stabile Haut gebildet und das quellbare hydrogelbildende Polymer im Becherglas bleibt, "sehr gut" bedeutet, dass sich das quellbare hydrogelbildende Polymer vollständig ausschütten lässt, und die Werte dazwischen bedeuten, dass an der Becherglaswand ein Rand aus quellbare hydrogelbildende Polymer verbleibt.

Beispiele

Beispiele 1 und 2:

In einem Lödige Labormischer wurde Grundpolymer ASAP 500 Z durch Aufsprühen von 3,16 Gew.-% Isopropanol/Wasser (30:70) und 0,085 Gew.-% 2-Oxazolidinon, jeweils bezogen auf das Grundpolymer, und anschließendem Aufheizen auf 175°C für 120 Minuten nachvernetzt. Dabei wurden ggf. dendritische Polymere hinzugefügt. Anschließend wurde das erhaltene Polymer bei 850$\mu$m abgesiebt und von Klumpen befreit. Die Produkte wurden getestet und auf Feinstaub (<10$\mu$m) mittels Laser-Fallrohrmethode analysiert. Dieselben Produkte wurden mittels einer Rollmühle mechanisch degradiert und nochmals auf den Staubgehalt geprüft.

Tabelle 1:

| Versuch | Additiv | CRC [g/] | AUL 0.7 [g/g] | SFC [$10^7 cm^3 s/g$] | FLR [g/s] | ASG [g/ml] | Staub vor Mühle [ppm] | Staub nach Mühle [ppm] | Anti-Caking Test |
|---|---|---|---|---|---|---|---|---|---|
| 1 | | 30,9 | 23,3 | 45 | 10,4 | 0,68 | 2 | 26 | sehr schlecht |
| 2 | 0,5 Gew.-% Boltorn® H40 | 29,7 | 22,3 | 33 | 9,4 | 0,64 | 1 | <10 | n.d. |

## Patentansprüche

1. Quellbares hydrogelbildendes Polymer, enthaltend bis zu 10 Gew.-%, bezogen auf das quellbare hydrogelbildende Polymer, mindestens ein hydrophiles Polymer mit dendritischer Struktur.

2. Polymer gemäß Anspruch 1, wobei das quellbare hydrogelbildende Polymer mindestens 0,005 Gew.-% hydrophiles Polymer mit dendritischer Struktur enthält.

3. Polymer gemäß Anspruch 1 oder 2, wobei das hydrophile Polymer mit dendritischer Struktur ein Polyester aus einem Polyol und 2,2-Dimethylolpropionsäure ist.

4. Polymer gemäß einem der Ansprüche 1 bis 3, wobei das hydrophile Polymer mit dendritischer Struktur ein Polypropylenimin, ein Polyamidoamin oder ein Polyesteramid ist.

5. Polymer gemäß einem der Ansprüche 1 bis 4, welches zusätzlich ein pulverförmiges und/oder staubförmiges Additiv enthält.

6. Polymer gemäß Anspruch 5, wobei das Additiv ein Metallsalz, eine pyrogene Kieselsäure, ein Polysaccharid, ein nichtionischs Tensid, ein Wachs und/oder Diatomeenerde ist.

7. Polymer gemäß einem der Ansprüche 5 oder 6, wobei das Additiv in Form von Mikrohohlkugeln vorliegt, deren Durchmesser von 1 bis 1000 μm beträgt, wobei die Wanddicke der Mikrohohlkugeln 1 bis 10 % des Durchmessers ausmacht.

8. Polymer gemäß einem der Ansprüche 1 bis 7, wobei der Anteil an Partikeln mit einem Durchmesser von weniger als 10 μm weniger als 50 Gew.-ppm beträgt.

9. Polymer gemäß einem der Ansprüche 1 bis 8, **dadurch gekennzeichnet, dass** der Anteil an Partikeln mit einem Durchmesser von weniger als 10 μm nach mechanischer Belastung weniger als 50 Gew.-ppm beträgt.

10. Verfahren zur Herstellung eines quellbaren hydrogelbildenden Polymers, gemäß einer der Ansprüche 1 bis 9, **dadurch gekennzeichnet, dass** man ein getrocknetes, wasserabsorbierendes Hydrogel mit mindestens einem hydrophilen Polymer mit dendritischer Struktur vermischt.

11. Verfahren gemäß Anspruch 10, **dadurch gekennzeichnet, dass** man als hydrophiles Polymer mit dendritischer Struktur ein Polyester aus einem Polyol und 2,2-Dimethylolpropionsäure einsetzt.

12. Verfahren gemäß Anspruch 10, **dadurch gekennzeichnet, dass** man als hydrophiles Polymer mit dendritischer Struktur ein Polypropylenimin, ein Polyamidoamin oder ein Polyesteramid einsetzt.

13. Verfahren gemäß einem der Ansprüche 10 bis 12, **dadurch gekennzeichnet, dass** man die Nachbehandlung zusammen mit einer Oberflächennachvernetzung durchführt.

14. Verfahren gemäß Anspruch 13, **dadurch gekennzeichnet, dass** das Lösungsmittel, welches mindestens einen Oberflächennachvernetzer enthält, ein Gemisch aus Isopropanol und Wasser ist.

15. Verwendung der Polymere gemäß einem der Ansprüche 1 bis 9 zur Absorption von Blut und/oder Köperflüssigkeiten.

16. Verwendung gemäß Anspruch 15 zur Absorption von Urin.

17. Hygieneartikel, enthaltend ein Polymer gemäß einem der Ansprüche 1 bis 9.

## Claims

1. Swellable hydrogel-forming polymer comprising up to 10% by weight, based on the swellable hydrogel-forming polymer, of at least one hydrophilic polymer of dendritic structure.

2. Polymer according to claim 1 wherein said swellable hydrogel-forming polymer comprises at least 0.005% by weight of hydrophilic polymer of dendritic structure.

3. Polymer according to claim 1 or 2 wherein said hydrophilic polymer of dendritic structure is a polyester formed from a polyol and 2,2-dimethylolpropionic acid.

4. Polymer according to any one of claims 1 to 3 wherein said hydrophilic polymer of dendritic structure is a polypropyleneimine, a polyamidoamine or a polyesteramide.

5. Polymer according to any one of claims 1 to 4 further comprising a powdery and/or dusty additive.

6. Polymer according to claim 5 wherein said additive is a metal salt, a fumed silica, a polysaccharide, a nonionic surfactant, a wax and/or diatomaceous earth.

7. Polymer according to either of claims 5 and 6 wherein said additive is present in the form of hollow microspheres which are from 1 to 1000 μm in diameter and whose wall thickness comprises from 1% to 10% of said diameter.

8. Polymer according to any one of claims 1 to 7 comprising less than 50 weight ppm of particles less than 10 μm in diameter.

9. Polymer according to any one of claims 1 to 8 comprising less than 50 weight ppm of particles less than 10 μm in diameter after exposure to mechanical stress.

10. A process for preparing a swellable hydrogel-forming polymer according to any one of claims 1 to 9, which comprises mixing a dried, water-absorbing hydrogel with at least one hydrophilic polymer of dendritic structure.

11. The process according to claim 10 wherein said hydrophilic polymer of dendritic structure is a polyester formed from a polyol and 2,2-dimethylolpropionic acid.

12. The process according to claim 10 wherein said hydrophilic polymer of dendritic structure is a polypropyleneimine, a polyamidoamine or a polyesteramide.

13. The process according to any one of claims 10 to 12 wherein said aftertreating is carried out together with a surface-postcrosslinking operation.

14. The process according to claim 13 wherein the solvent which comprises at least one surface postcrosslinker is a mixture of isopropanol and water.

15. The use of the polymers according to any one of claims 1 to 9 for absorbing blood and/or body fluids.

16. The use according to claim 15 for absorbing urine.

17. Hygiene articles comprising a polymer according to any one of claims 1 to 9.

## Revendications

1. Polymère expansible, formant un hydrogel, contenant jusqu'à 10 % en poids, par rapport au polymère expansible formant un hydrogel, d'au moins un polymère hydrophile à structure dendritique.

2. Polymère suivant la revendication 1, dans lequel le polymère expansible, formant un hydrogel, contient au moins 0,005 % en poids de polymère hydrophile à structure dendritique.

3. Polymère suivant la revendication 1 ou 2, dans lequel le polymère hydrophile à structure dendritique est un polyester à base d'un polyol et d'acide 2,2-diméthylolpropionique.

4. Polymère suivant l'une des revendications 1 à 3, dans lequel le polymère hydrophile à structure dendritique est une polypropylèneimine, une polyamidoamine ou un polyesteramide.

5. Polymère suivant l'une des revendications 1 à 4, qui contient en supplément un additif en forme de poudre et/ou de poussière.

6. Polymère suivant la revendication 5, dans lequel l'additif est un sel métallique, un acide silicique pyrogène, un polysaccharide, un agent tensioactif non ionique, une cire et/ou de la terre de diatomées.

7. Polymère suivant l'une des revendications 5 et 6, dans lequel l'additif se présente sous la forme de microbilles creuses, dont le diamètre est de 1 à 1000 μm, l'épaisseur de paroi des microbilles creuses représentant 1 à 10 % du diamètre.

8. Polymère suivant l'une des revendications 1 à 7, dans lequel la fraction de particules ayant un diamètre de moins de 10 μm est inférieure à 50 ppm en poids.

9. Polymère suivant l'une des revendications 1 à 8, **caractérisé en ce que** la fraction de particules ayant un diamètre de moins de 10 μm est, après sollicitation mécanique, inférieure à 50 ppm en poids.

10. Procédé de préparation d'un polymère expansible, formant un hydrogel, suivant l'une des revendications 1 à 9, **caractérisé en ce qu'**on mélange un hydrogel absorbant l'eau, séché, avec au moins un polymère hydrophile à structure dendritique.

**11.** Procédé suivant la revendication 10, **caractérisé en ce que**, comme polymère hydrophile à structure dendritique, on met en oeuvre un polyester à base d'un polyol et d'acide 2,2-diméthylolpropionique.

**12.** Procédé suivant la revendication 10, **caractérisé en ce que**, comme polymère hydrophile à structure dendritique, on met en oeuvre une polypropylèneimine, une polyamidoamine ou un polyesteramide.

**13.** Procédé suivant l'une des revendications 10 à 12, **caractérisé en ce qu'**on effectue le traitement ultérieur conjointement à une postréticulation de surface.

**14.** Procédé suivant la revendication 13, **caractérisé en ce que** le solvant, qui contient au moins un agent de postréticulation de surface, est un mélange d'isopropanol et d'eau.

**15.** Utilisation des polymères suivant l'une des revendications 1 à 9, pour l'absorption de sang et/ou de liquides corporels.

**16.** Utilisation suivant la revendication 15, pour l'absorption d'urine.

**17.** Articles d'hygiène, contenant un polymère suivant l'une des revendications 1 à 9.